# EUROPÄISCHE PATENTSCHRIFT

(11) **EP 0 945 128 B1**
(45) Veröffentlichungstag und Bekanntmachung des Hinweises auf die Patenterteilung: **06.10.2004**
(21) Anmeldenummer: 99101745.0
(22) Anmeldetag: 11.02.1999
(51) Int. Cl.: A61K 7/48, A61K 7/00

(54) **Verwendung von Flavonen, Flavanonen bzw. Flavonoiden zum Schutze von Ascorbinsäure und/oder Ascorbylverbindungen gegen Oxidation**
Use of flavones, flavanones, or flavonoides for the protection of ascorbic acid and/or ascorbylbonds against oxidation
Utilisation de flavones, flavanones, ou de flavonoides pour la protection de l'acide ascorbique et/ou de liaisons de type ascorbyle contre l'oxydation

(30) Priorität: 24.02.1998 DE 19807774
(43) Veröffentlichungstag der Anmeldung: 29.09.1999
(73) Patentinhaber: Beiersdorf Aktiengesellschaft, 20245 Hamburg (DE)
(72) Erfinder: Kruse, Inge, 20146 Hamburg (DE); Schönrock, Uwe, Dr., 23866 Nahe (DE)

(56) Entgegenhaltungen:
- EP-A- 0 461 827
- DE-A- 4 444 238
- STN, Karlsruhe, DE, Datenbank Chemical Abstracts, BD 70, AN=36541 XP002119509
- SHRIKHANDE ET AL. : "Effect of flavonols on ascorbic acid and anthocyanin stability in model systems" J. FOOD SCIENCE, Bd. 39, Nr. 4, 1974, Seiten 904-906, XP002120098 Chicago, USA
- STN, Karlsruhe, DE, Datenbank Medline, AN=60039601 XP002119510
- STN, Karlsruhe, DE, Datenbank Chemical Abstracts, BD 68, AN=62645 XP002119511

## Beschreibung

Die vorliegende Erfindung betrifft die Verwendung von α- Glucosylrutin zum Schutze von Ascorbinsäure und/oder generell Ascorbylverbindungen gegen Oxidation, insbesondere in kosmetischen und dermatologischen Zubereitungen. Bevorzugt betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die antioxidativ wirksame Wirkstoffkombinationen sind bevorzugt solche, welche in hautpflegenden kosmetischen oder dermatologischen Zubereitungen eingesetzt werden. Die Erfindung findet Auwendung für kosmetische und dermatologische Zubereitungen, solche Antioxidantien enthaltend.

In einer bevorzugten Ausführungsform, die vorliegende Erfindung findet Auwendung für kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin, die vorliegende Erfindung findet Auwendung für Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung findet Auwendung für Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikatische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Durch all diese Einflüsse ist die Zelle dem sogenannten oxidativen Streß ausgesetzt, unter welchem verstanden wird die Belastung der lebenden Zelle durch Anreicherung zellschädigender oxidierter Verbindungen wie beispielsweise Lipid-Hydroperoxide, Wasserstoffperoxid oder reaktive Sauerstoffverbindungen wie Singulett-Sauerstoff, Hydroxyl, Hyperoxid-Anionen. Der oxidative Streß kann z. B. durch Strahlungs-Einwirkung, Xenobiotika, Schwermetall-lonen oder andere Ursachen induziert werden. Er spielt eine Rolle bei der Entstehung einiger Erkrankungen.

Zwar besitzt der Körper in begrenztem Maße Abwehrmechanismen gegen den oxidativen Streß. So können mit Hilfe verschiedener Enzym-Aktivitäten [z. B. Superoxid-Dismutase, Peroxidasen wie Glutathionperoxidase (s. Glutathion), Katalase, Caeruloplasmin] das Auftreten reaktiver Radikale verhindert werden. Dies reicht aber oftmals nicht aus und muß, sei es auf kosmetischem oder medizinischem Niveau, Unterstützung erfahren.

Um oxidativem Streß vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar.

Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Antioxidantien sind Substanzen, welche Oxidationsprozesse verhindern bzw. welche die Autoxidation ungesättigte Verbindungen enthaltender Fette verhindern. Antioxidantien, welche auch auf dem Gebiete der Kosmetik und der Pharmazie Verwendung finden, sind beispielsweise α-Tocopherol, insbesondere in Form des α-Tocopherylacetats, Sesamol, Gallensäurederivate, Butylhydroxyanisol und Butylhydroxytoluol.

Auch aus dem Grunde, solchen Reaktionen vorzubeugen, können kosmetischen Formulierungen zusätzlich Antioxidantien und/oder Radikalfänger einverleibt werden.

Antioxidantien sind chemische Substanzen unterschiedlichster Struktur, die unerwünschte, durch Sauerstoff-Einwirkungen und andere oxidative Prozesse bedingte Veränderungen in den zu schützenden Stoffen hemmen oder verhindern. Antioxidantien werden insbesondere eingesetzt, um organische Produkte zu schützen, namentlich Fette und Öle, aber auch Wirk- und Zusatzstoffe wie beispielsweise Aromastoffe und dergleichen. Als Antioxidantien wirksam sind beispielsweise durch sterisch hindernde Gruppen substituierte Phenole, Hydrochinone, Brenzcatechine und aromatische Amine sowie deren Metall-Komplexe. Für Fette, Lebensmittel und insbesondere auch kosmetische und dermatologische Zubereitungen eignen sich Tocopherol, Butylhydroxyanisol (BHA), Butylhydroxytoluol (BHT), Octyl- und Dodecylgallat sowie Ascorbin-, Milch-, Citronen- und Weinsäure sowie deren Salze

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

An sich vorzügliches Antioxidantien werden gewählt aus der Gruppe der Ascorbinsäure und der Ascorbylverbindungen.

L-Ascorbinsäure {(R)-5-[(S)-1,2-Dihydroxyethyl]-3,4-dihydroxy-5-H-furan-2-on, Vitamin C} zeichnet sich durch die Strukturformel 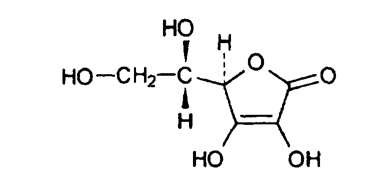 aus. Sie ist leicht löslich in Wasser, gut löslich in Alkohol, unlöslich in Ether, Petrolether, Chloroform, Benzol sowie in Fetten u. fetten Ölen. Ascorbinsäure ist ein Endiol und wirkt als Redukton stark reduzierend. Ascorbinsäure ist wärmeempfindlich und wird insbesondere in Gegenwart von Schwermetallspuren sowie in alkalischem Milieu durch Licht und Luftsauerstoff zersetzt, in reinem, trockenem Zustand ist sie dagegen relativ beständig gegen Licht, Luft und Wärme.

In kosmetischen und dermatologischen Zubereitungen werden anstatt der Ascorbinsäure oftmals Ascorbylverbindungen, bevorzugt Ascorbylester von Fettsäuren, besonders bevorzugt Ascorbylpalmitat eingesetzt, da die Empfindlichkeit dieser Verbindungen auf oxidativen Einfluß gegenüber der Ascorbinsäure stark herabgesetzt ist und diese Verbindungen zumeist besser öllöslich sind, was galenische Vorteile bieten kann.

Ascorbylverbindungen im engeren Sinne sind insbesondere die Ascorbylester der allgemeinen Struktur 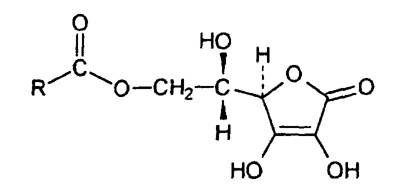 wobei R einen verzweigten oder unverzweigten Alkylrest mit bis zu 25 Kohlenstoffatomen darstellen kann.

Eine Aufgabe der vorliegenden Erfindung war, die Nachteile des Standes der Technik zu beseitigen. Insbesondere sollten Wirkstoffe bzw. Zubereitungen, solche Wirkstoffe enthaltend, zur Verfügung gestellt werden, bei deren Verwendung die Schädigung der Haut und/oder des Haares durch oxidativen Einfluß zumindest gemindert, wenn nicht gänzlich verhindert werden können.

Eine weitere Aufgabe der vorliegenden Erfindung bestand darin, kosmetische Zubereitungen zur Verfügung zu stellen, welche vor oder nach Behandlung des Haars mit Haarfärbezubereitungen, selbst solcher mit einem Gehalt an starken Oxidationsmitteln wie z.B. Wasserstoffperoxid, deren schädigenden Oxidationswirkung entgegenwirken.

Insbesondere sollten Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung gestellt werden.

In einer besonderen Aufgabenstallung sollten Wege aufgefunden werden, Ascorbylverbindungen, insbesondere Vitamin C und Vitamin-C-Ester gegen schädlichen oxidativen Einfluß zu schützen, und zwar bevorzugt in kosmetischen oder dermatologischen Zubereitungen.

Die Verwendung von Flavonen bzw. Flavonoiden in der Kosmetik bzw. Dermatologie ist an sich bekannt. So beschreibt die DE-OS 44 44 238 Kombinationen von Zimtsäurederivaten und Flavonglycosiden, beispielsweise α-Glycosylrutin als Antioxidantien und als Wirkstoffe gegen andere Indikationen.

Es war indes überraschend und für den Fachmann nicht vorherzusehen, daß die Verwendung von α-Glucosylrutin zum Schutze mindestens eines Wirkstoffes, gewählt aus der Gruppe der Ascorbinsäure und der Ascorbylverbindungen gegen Oxidation, und zwar insbesondere zum Schutze gegen Oxidation in kosmetischen oder dermatologischen Zubereitungen, den Nachteilen des Standes der Technik abhelfen.

Das Dokument Chemical Abstract, Band 70, AN=36541, beschreibt die Verwendung der Flavonoide Quercitin, Dihydroquercitin und Kaempferol zum Schutze von Ascorbinsäure.

α-Glucosylrutin zeichnet sich durch folgende Struktur aus:

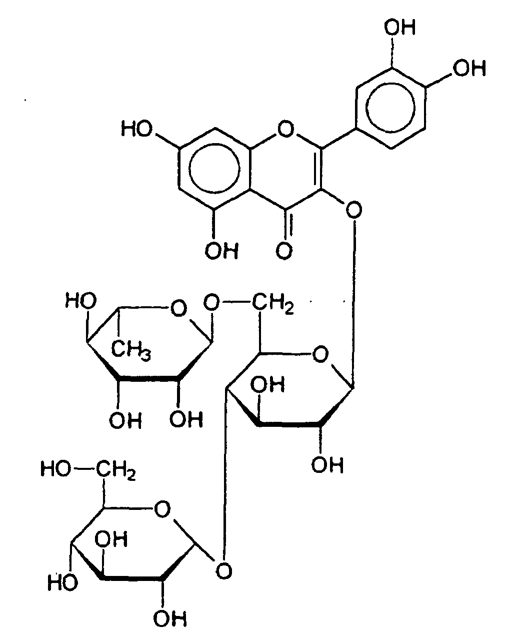

Das α-Glucosylrutin wird erfindungsgemäß in kosmetischen oder dermatologischen Zubereitungen zu 0,001 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Die Ascorbylverbindung oder die Ascorbylverbindungen, insbesondere Vitamin C, ist bzw. sind erfindungsgemäß vorteilhaft in kosmetischen oder dermatologischen Zubereitungen bevorzugt zu 0,001 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht

der Zubereitungen, enthalten.

Die erfindungsgemäße Kombination aus α-Glucosylrutin und mindestens einer Ascorbylverbindung, insbesondere Vitamin C, wird im Rahmen dieser Schrift auch kollektiv als erfindungsgemäßer Wirkstoff" oder erfindungsgemäß verwendeteter Wirkstoff" oder erfindungsgemäß verwendetete Wirkstoffkombination" bezeichnet bzw. mit sinnverwandten Bezeichnungen belegt.

Die Schrift JP-OS Hei-06-138,941 beschreibt zwar orale Zubereitungen mit einem Gehalt an wasserlöslichen Glycosiden, welche beispielsweise gewählt werden können aus der Gruppe α-Glucosylrutin, α-Glucosylmyricetin, α-Glucosylisoquercitrin und α-Glucosylquercitrin. Die Schrift JP-OS Hei-04-363,395 beschreibt ein Verfahren, die Zersetzung von Parfümbestandteilen zu verhindern, welche sich unter anderem durch einen Zusatz an α-Glucosylrutin zu den entsprechenden Zubereitungen auszeichnet. Ferner beschreiben die Schriften EP-OS 586 303 und EP-OS 595 694 die Verwendung von Flavonoïden als Antioxidantien bzw. Lichtschutzsubstanzen in Kosmetika.

Kein Hinweis ist diesen Schriften aber zu entnehmen, welcher in die Richtung der vorliegenden Erfindung weisen könnte.

Es war für den Fachmann daher nicht vorauszusehen gewesen, daß die erfindungsgemäß verwendeten Wirkstoffkombinationen bzw. kosmetische oder dermatologische Zubereitungen, solche enthaltend
- besser als Antioxidans wirken
- besser als Radikalfänger wirken
- besser die Bindung von schädlichen Photoprodukten an Lipide, DNS und Proteine verhindern
- besser gegen die Hautalterung wirken
- besser die Haut gegen Photoreaktionen schützen
- besser entzündlichen Reaktionen vorbeugen würde
als die Wirkstoffe, Wirkstoffkombinationen und Zubereitungen des Standes der Technik. Ferner war nicht vorauszusehen gewesen, daß die erfindungsgemäß verwendeten Wirkstoffkombinationen in kosmetischen oder dermatologischen Zubereitungen höhere Stabilität aufweist als die jeweils einzeln verwendeten Wirkstoffe, was insbesondere die Ascorbylverbindungen und ganz besonders das Vitamin C betrifft.

Erfindungsgemäß sind daher die Verwendung von Wirkstoffkombinationen aus α-Glucosylrutin wird und Ascorbinsäure und/oder Ascorbylverbindungen als Antioxidans sowie seine Verwendung zur Bekämpfung und/oder Prophylaxe der durch oxidative Beanspruchung hervorgerufenen Hautalterung und entzündlicher Reaktionen.

Als besonders vorteilhafte Ausführungsform der vorliegenden Erfindung wird ferner angesehen die Verwendung von Wirkstoffkombinationen aus α-Glucosylrutin wird und Ascorbinsäure und/oder Ascorbylverbindungen zur Bekämpfung und/oder Prophylaxe des oxidativen Stresses.

Die erfindungsgemäßen kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an erfindungsgemäß verwendeten Wirkstoffkombinationen.

Es ist erfindungsgemäß bevorzugt, den erfindungsgemäß verwendeten Wirkstoffkombinationen bzw. kosmetischen oder dermatologischen Zubereitungen, solche Wirkstoffkombinationen enthaltend Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch vorteilhaft, Isoquercitrin in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, erfindungsgemäß verwendete Wirkstoffkombinationen in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können erfindungsgemäß verwendete Wirkstoffkombinationen auch mit anderen Antioxidantien und/oder Radikalfängern kombiniert werden.

Vorteilhaft werden solche Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonornethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoäsäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein. Die erfindungsgemäß verwendeten Wirkstoffkombinationen können kombiniert werden mit mindestens einem UVB-Filter als Antioxidans bzw. eingesetet werden als Kombination der erfindungsgemäß verwendeten Wirkstoffkombinationen mit mindestens einem UVB-Filter als Antioxidans in einer kosmetischen oder dermatologischen Zubereitung.

Es kann auch von Vorteil sein, die erfindungsgemäß verwendeten Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind möglich. Es können die für die UVB-Kombination verwendeten Mengen eingesetzt werden.

Die Erfindung findet auch Auwendung in die Verwendung einer Kombination von erfindungsgemäß verwendeten Wirkstoffkombinationen mit mindestens einem UVA-Filter als Antioxidans bzw. die Verwendung einer Kombination der erfindungsgemäßen Wirkstoffkombinationen mit mindestens einem UVA-Filter als Antioxidans in einer kosmetischen oder dermatologischen Zubereitung.

Die Erfindung findet auch Auwendung in die Verwendung einer Kombination aus erfindungsgemäß verwendeten Wirkstoffkombinationen mit mindestens einem UVA-Filter und mindestens einem UVB-Filter als Antioxidans bzw. die Verwendung einer Kombination aus Isoquercitrin mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an erfindungsgemäß verwendeten Wirkstoffkombinationen können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind möglich. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, die erfindungsgemäß verwendeten Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäß verwendete Wirkstoffkombinationen in wirksamer Konzentration. Die Menge der verwendeten Polymeren liegt z.B. zwischen 0,1 und 10 Gew.-%, bevorzugt zwischen 0,1 und 3 Gew.-%.

Kosmetische Zubereitungen zur Behandlung und Pflege der Haare, die die erfindungsgemäß verwendeten Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an Isoquercitrin und dafür üblicherweise verwendeten Lösungsmitteln, bevorzugt Wasser, noch organische Verdickungsmittel, z.B. Gummiarabikum, Xanthangummi, Natriumalginat, CelluloseDerivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge an Isoquercitrin in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate

Kosmetische Zubereitungen, die kosmetische Reinigungszubereitungen für die Haut darstellen, können in flüssiger oder fester Form vorliegen. Sie enthalten neben erfindungsgemäß verwendeten Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an Isoquercitrin vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die vorliegende Erfindung findet auch Anwendung in ein kosmetisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an erfindungsgemäß verwendeten Wirkstoffkombinationen enthält, in ausreichender Menge auf die Haut oder Haare aufbringt.

die vorliegenden Erfindung findet auch Anwendung in ein Verfahren zum Schutze kosmetischer oder dermatologischer Zubereitungen gegen Oxidation oder Photooxidation, wobei diese Zubereitungen z.B. Zubereitungen zur Behandlung und Pflege der Haare darstellen, insbesondere Haarfärbemittel, Haarlacke, Shampoonierungsmittel, Farbshampoonierungsmittel, ferner Schminkprodukte wie z.B. Nagellacke, Lippenstifte, Teintgrundlagen, Wasch- und Duschzubereitungen, Cremes zur Behandlung oder Pflege der Haut oder um sämtliche anderen kosmetischen Zubereitungen handelt, deren Bestandteile Stabilitätsprobleme aufgrund von Oxidation bzw. Photooxidation bei der Lagerung mit sich bringen können, dadurch gekennzeichnet, daß die kosmetischen Zubereitungen einen wirksamen Gehalt an Isoquercitrin aufweisen.

Vorzugsweise beträgt die Menge an erfindungsgemäß verwendeten Wirkstoffkombinationen in diesen Zubereitungen 0,01 - 10 Gew.-%, bevorzugt 0,05 - 5 Gew.-%, insbesondere 0,1 - 2,0 Gew.-%, bezogen auf das Gesamtgewicht der Zubereitungen.

Die Erfindung findet auch Anwendung in Verfahren zur Herstellung der erfindungsgemäßen kosmetischen Mittel, das dadurch gekennzeichnet ist, daß man in an sich bekannter Weise erfindungsgemäßen Wirkstoffkombinationen in kosmetische und dermatologische Formulierungen einarbeitet.

Obwohl sich die erfindungsgemäß erzielten kosmetischen oder dermatologischen Zubereitungen und die darin befindlichen Wirkstoffkombinationen durch erhöhte Stabilität gegenüber oxidativem Einfluß auszeichnen, sind doch Lagerungsformen bevorzugt, in welchen ein verminderter Zutritt von Luftsauerstoff gegeben ist. So ist beispielsweise die Befüllung unter Inertgas, insbesondere Stickstoff, vorteilhaft. Als vorteilhafte Verpackung haben sich insbesondere Aluminiumtuben herausgestellt.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

| O/W Crème | |
|---|---|
| Glycerylstearat | Gew.-% 5,00 |
| Cetylalkohol | 5,00 |
| Isopropylpalmitat | 7,00 |
| Cyclomethicon | 5,00 |
| Ascorbinsäure | 3,00 |
| α-Glucosylrutin | 0,30 |
| NaOH 45 %-ig | 1,00 |
| Butylenglycol | 3,00 |
| Na₂H₂EDTA | 0,20 |
| Farbstoffe, Parfum, Konservierungsmittel | q.s. |
| Wasser | ad 100,00 |

### Beispiel 2

| O/W Lotion | |
|---|---|
| | Gew.-% |
| Steareth-20 | 3,00 |
| Cetylalkohol | 3,00 |
| Cyclomethicon | 6,00 |
| Carbomer | 0,60 |
| Na₂H₂EDTA | 0,20 |
| Butylenglycol | 3,00 |
| NaOH 45 %-ig | 0,40 |
| Ascorbinsäure | 0,50 |
| α-Glucosylrutin | 0,10 |
| Farbstoffe, Parfum, Konservierungsmittel | q.s. |
| Wasser | ad 100,00 |

### Beispiel 3

| W/O Crème | |
|---|---|
| | Gew.-% |
| Polyglyceryl-2-dipolyhydroxystearat | 5,00 |
| Caprylic/Capric Triglyceride | 15,00 |
| Butylenglycol | 3,00 |
| Na₂H₂EDTA | 0,20 |
| MgSO₄ | 0,70 |
| NaOH 45 %-ig | 0,32 |
| Ascorbinsäure | 1,00 |
| α-Glucosylrutin | 0,20 |
| Farbstoffe, Parfum, Konservierungsmittel | q.s. |
| Wasser | ad 100,00 |

### Beispiel 4

| **Beispiel 4** O/W-Gel | |
|---|---|
| | Gew.-% |
| Xanthangummi | 2,00 |
| Butylenglycol | 3,00 |
| Na₂H₂EDTA | 0,20 |
| NaOH 45 %-ig | 0,32 |
| Ascorbinsäure | 1,00 |
| α-Glucosylrutin | 0,20 |
| Farbstoffe, Parfum, Konservierungsmittel | q.s. |
| Wasser | ad 100,00 |

### Beispiel 5

| O/W-Haarkur | |
|---|---|
| | Gew.-% |
| Bis-Diglycerylpolyacyladipat-2 | 3,00 |
| Behenylalkohol | 4,00 |
| Butylenglycol | 3,00 |
| Cetrimoniumchlorid | 5,00 |
| Citronensäure | 0,50 |
| Na₂H₂EDTA | 0,20 |
| NaOH 45 %-ig | 0,16 |
| Ascorbinsäure | 0,50 |
| α-Glucosylrutin | 0,10 |
| Farbstoffe, Parfum, Konservierungsmittel | q.s. |
| Wasser | ad 100,00 |

## Patentansprüche

1. Verwendung von α-Glucosylrutin, zum Schutze mindestens eines Wirkstoffes, gewählt aus der Gruppe der Ascorbinsäure und der Ascorbylverbindungen gegen Oxidation, **dadurch gekennzeichnet, daß** das α-Glucosylrutin in kosmetischen oder topischen dermatologischen Zubereitungen in Konzentrationen von 0.001 - 10 Gew.-%, bevorzugt 0,05 - 5 Gew.-%, insbesondere 0,1 - 2,0 Gew.-% bezogen auf das Gesamtgewicht der Zubereitungen, vorliegt.

2. Verwendung nach Anspruch 1, **dadurch gekennzeichnet, daß** daß der oder die Wirkstoffe, gewählt aus der Gruppe der Ascorbinsäure und der Ascorbylverbindungen in kosmetischen oder topischen dermatologischen Zubereitungen in Konzentrationen von 0,001 - 10 Gew.-%, bevorzugt 0,05 - 5 Gew.-%, insbesondere 0,1 - 2,0 Gew.-% bezogen auf das Gesamtgewicht der Zubereitungen, vorliegt.

3. Verwendung nach Anspruch 2, **dadurch gekennzeichnet, daß** die kosmetischen oder dermatologischen Zubereitungen zusätzlich einen oder mehrere Komplexbildner enthalten.

4. Verwendung nach Anspruch 1, **dadurch gekennzeichnet, daß** der oder die Komplexbildner gewählt wird oder werden aus der Gruppe bestehend aus Weinsäure und deren Anionen, Citronensäure und deren Anionen, Aminopolycarbonsäuren und deren Anionen (wie beispielsweise Ethylendiamintetraessigsäureund deren Anionen, Nitrilotriessigsäure und deren Anionen, Hydroxyethylendiaminotriessigsäure und deren Anionen, Diethylenaminopentaessigsäure und deren Anionen, trans-1,2-Diaminocyclohexantetraessigsäure und deren Anionen).

5. Verwendung nach Anspruch 1, **dadurch gekennzeichnet, daß** der oder die Komplexbildner sind in den kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten ist oder sind.

## Claims

1. Use of α-glucosylrutin for protecting at least one active ingredient chosen from the group consisting of ascorbic acid and ascorbyl compounds from oxidation **characterized in that** the α-glucosylrutin is present in cosmetic or topical dermatological preparations in concentrations of 0.001-10% by weight, preferably 0.05-5% by weight, in particular 0.1-2.0% by weight, based on the total weight of the preparations.

2. Use according to Claim 1, **characterized in that** that [sic] the active ingredient(s) chosen from the group consisting of ascorbic acid and ascorbyl compounds is/are present in cosmetic or topical dermatological preparations in concentrations of 0.001-10% by weight, preferably 0.05-5% by weight, in particular 0.1-2.0% by weight, based on the total weight of the preparation.

3. Use according to Claim 2, **characterized in that** the cosmetic or dermatological preparations additionally comprise one or more complexing agents.

4. Use according to Claim 1 [sic], **characterized in that** the complexing agent(s) is/are chosen from the group consisting of tartaric acid and anions thereof, citric acid and anions thereof, aminopolycarboxylic acids and anions thereof (such as, for example, ethylenediaminetetraacetic acid and anions thereof, nitrilotriacetic acid and anions thereof, hydroxyethylenediaminotriacetic acid and anions thereof, diethyleneaminopentaacetic acid and anions thereof, and trans-1,2-diaminocyclohexanetetraacetic acid and anions thereof).

5. Use according to Claim 1 [sic], **characterized in that** the complexing agent(s) are [sic] is/are present in the cosmetic or dermatological preparations preferably in amounts from 0.01% by weight to 10% by weight, preferably from 0.05% by weight to 5% by weight, particularly preferably 0.1-2.0% by weight, based on the total weight of the preparations.

## Revendications

1. Utilisation d'α-glucosylrutine pour la protection contre l'oxydation d'au moins une substance active choisie dans le groupe constitué par l'acide ascorbique et les composés ascorbyle, **caractérisée en ce que** l'α-glucosylrutine se trouve dans des préparations cosmétiques ou dermatologiques topiques à des concentrations de 0,001 à 10 % en poids, de préférence de 0,05 à 5 % en poids, en particulier de 0,1 à 2,0 % en poids, par rapport au poids total des préparations.

2. Utilisation selon la revendication 1, **caractérisée en ce que** la ou les substances actives choisies dans le groupe de l'acide ascorbique et des composés ascorbyle se trouve(nt) dans des préparations cosmétiques ou dermatologiques topiques à des concentrations de 0,001 à 10 % en poids, de préférence de 0,05 à 5 % en poids, en particulier de 0,1 à 2,0 % en poids, par rapport au poids total des préparations.

3. Utilisation selon la revendication 2, **caractérisée en ce que** les préparations cosmétiques ou dermatologiques contiennent en outre un ou plusieurs complexants.

4. Utilisation selon la revendication 1, **caractérisée en ce que** le ou les complexants est ou sont choisis dans le groupe constitué par l'acide tartrique et ses anions, l'acide citrique et ses anions, des acides aminopolycarboxyliques et leurs anions (comme par exemple l'acide éthylènediaminetétraacétique et ses anions, l'acide nitrilotriacétique et ses anions, l'acide hydroxyéthylènediaminotriacétique et ses anions, l'acide diéthylèneaminopentaacétique et ses anions, l'acide trans-1,2-diaminocyclohexanetétraacétique et ses anions).

5. Utilisation selon la revendication 1, **caractérisée en ce que** le ou les complexants est ou sont contenus dans les préparations cosmétiques ou dermatologiques de préférence à raison de 0,01 % en poids à 10 % en poids, de préférence de 0,05 % en poids à 5 % en poids, de façon particulièrement préférée de 0,1 à 2,0 % en poids, par rapport au poids total des préparations.
